# EUROPEAN PATENT APPLICATION

(11) **EP 2 269 544 A1**
(43) Date of publication of application: **05.01.2011**
(21) Application number: 09172912.9
(22) Date of filing: 13.10.2009
(51) Int. Cl.: A61F 7/00

(54) **Thermal treatment system and apparatus with biofeedback-driven protocol**

(30) Priority: 03.07.2009 EP 09164524
(71) Applicant: Dewaegenaere, Levi Emmerik A., 2970 s'-Gravenwezel (BE)
(72) Inventor: Dewaegenaere, Levi Emmerik A., 2970 s'-Gravenwezel (BE)
(74) Representative: Heunemann, Dieter

(57) **Abstract**

The invention provides for a thermal treatment device for generating a feedback-driven protocol for thermal treatment of a human or animal body part. The device comprises measurement means adapted to acquire data of a thermal treatment from the body part and means for adapting the thermal treatment of the body part on the basis of the data acquired. Further, a method for generating a feedback-driven protocol for thermal treatment of a human or animal body part is provided. Said method comprises the steps of acquiring data of a thermal treatment from the body part and adapting the thermal treatment of the body part on the basis of the data acquired (Fig. 1).

## Description

The invention relates to a thermal treatment device and method for generating a feedback-driven protocol for thermal treatment of a human or animal body part. In particular, data acquired from a thermal treatment of the body part are analyzed. On the basis of the acquired data, the treatment of the body part is then adapted to optimize the health-beneficial effect.

### FIELD OF THE INVENTION

The present invention is in the technical field of thermal treatment of the human body.

More particularly, the present invention is in the technical field of thermal treatment of the human or animal body by means of a device providing cooling and/or heating to the body utilizing a biofeedback to select and/or adapt a certain thermal treatment protocol.

### BACKGROUND OF THE INVENTION

Thermal treatment provides cooling and/or heating to the body in order to reach a beneficial health effect, such as, but not limited to pain reduction, fast swelling reduction and increased mobility.

Effective thermal treatment entails exchanging an amount of energy (heat or cold) with the body during a certain span of time and following a particular treatment sequence of treatment cycles. The optimal amount of energy exchanged depends on many factors that are difficult to control and will vary from patient to the patient and from disease to disease.

Accordingly, there is a need for an apparatus and system to overcome the problems in the prior art and to optimize the thermal treatment of a patient.

### OBJECTS OF THE INVENTION

The object of the present invention is to provide a thermal treatment apparatus and system optimizing the treatment of a patient.

It is a further object of this invention to provide a thermal treatment apparatus and system that automatically determines the optimum treatment protocol for a patient.

Further objects and advantages of the present invention will be disclosed and become apparent from the following description.

### SUMMARY OF THE INVENTION

The present invention relates to a thermal treatment device and method for generating a feedback-driven protocol for thermal treatment of a human or animal body part. More specific features for preferred embodiments are set out in the dependent claims.

In one embodiment, the present invention provides an apparatus and system comprising means to measure patient parameters resulting from the on-going thermal treatment and to feedback those parameters to the treatment device to control the on-going treatment. This system of biofeedback-driven protocol adaptation may yield a better and even optimal treatment for the specific patient.

According to the present invention, a thermal treatment device for generating a feedback-driven protocol for thermal treatment of a human or animal body part is provided. The device comprises measurement means adapted to acquire data of a thermal treatment from the body part and means for adapting the thermal treatment of the body part on the basis of the data acquired.

In a particular embodiment, the measurement means is adapted to determine the amount of thermal energy exchanged during the thermal treatment of the body part, on the basis of the acquired data.

In a particular embodiment, the means for adapting the thermal treatment is adapted to change the temperature and/or pressure and/or duration and/or duration/frequency of cycles of the thermal treatment and/or treatment content of each cycle of the thermal treatment.

In a particular embodiment, the device further comprises a storage means adapted to store the acquired data and to document the adapting of the thermal treatment.

For example, the documentation of the thermal treatment may be realized by generating a protocol and particularly safe/store the protocol on a processor unit which may be part of the thermal treatment device. A protocol may contain data and/or commands and/or sequences of commands and may be adapted to control the operation of the thermal treatment.

In a particular embodiment, the measurement means comprises at least one sensor adapted to determine a particular parameter of the body part.

In a particular embodiment, the particular parameter is at least one of the dimensions of the body part, the skin conductivity, the skin color, the skin temperature, the skin texture, the heart rate, the blood pressure, the blood saturation.

In a particular embodiment, the parameter determined by the sensor of the measurement means of the thermal treatment device is the dimensions of the body part and/or a parameter related to the thermal treatment, for example, flow rate, temperature, pressure pf the treatment. In this case, for example, a further device, e.g. a thermal exchange device, may comprise at least one sensor adapted to determine the body part related parameter(s), such as the skin conductivity, the skin color, the skin temperature, the skin texture, the heart rate, the blood pressure, the blood saturation.

According to the present invention, a system for generating a feedback-driven protocol for thermal treatment of a human or animal body part is provided. The system comprises a thermal treatment device as discussed above and a thermal exchange device connected to the thermal treatment device and adapted to provide a heating and/or a cooling of the body part.

In a particular embodiment, the system further comprises sensor means adapted to determine particular parameters of the body part.

As discussed above, the sensor may be adapted to determine the body part related parameter(s), such as the skin conductivity, the skin color, the skin temperature, the skin texture, the heart rate, the blood pressure, the blood saturation.

Additionally the system may comprise means for a controlled supply of medication, e.g., a certain pharmaceutical supplied by a drug pump, to the patient, wherein the system determines - via suitable sensor - the patient's response on the medication, namely the change of the body part related parameter(s). The system may provide a certain diagnosis based on the patient's response and/or modify the thermal treatment in a controlled manner in order to optimize the patient's treatment by means of a closed loop (feedback) control.

For example, the combination of treatment by the thermal treatment device in conjunction with other medical treatments, such as, but not limited to antibiotics, anti-inflammatory drugs, and chemotherapy drugs, can cause a positive synergistic treatment effect. In one embodiment, the thermal treatment device may by used to apply cryo-treatment to an area of the body (e.g, the arm) which is infected with a bacteria which is, for example, normally resistant to antibiotics (e.g. methicillin-resistant Staphylococcus aureus). The application of cryo-treatment in this way may cause a reduction in bacterial division and/or spread in the tissue, providing more time for the antibiotics to work effectively in the affected area. This may result in a favorable treatment outcome where amputation may have been the only other viable option.

Further, the invention disclosed herein could operate to monitor the patient's progress with regards to cryo-treatment in combination with other medical treatments and update the treatment protocol accordingly, for example, the choice of medicine and optimal dose rate to maximize pharmaceutical efficacy.

The temperature distribution data may be obtained by an IR (infrared) camera and may be displayed to the physician and/or used for feedback control of the thermal treatment. Furthermore, the time lapsed during the thermal treatment may also be taken into account when controlling the thermal treatment based on the determination of the various body part related parameter(s).

While the present invention discusses the use of biofeedback to adapt a treatment protocol, it is advantageous to disclose the optimal means of obtaining a biofeedback reading, which are preferably non-invasive. For example, the amount of thermal exchange between the thermal pad or Peltier thermal exchange element and skin can be determined by using a temperature sensitive surface between such thermal pad or Peltier thermal exchange element and skin. Alternatively, a measurement of power output to the fluid in the thermal pad or the Peltier thermal exchange element itself can provide an indirect determination of thermal exchange with a body. Alternatively, the amount of thermal exchange between a Peltier thermal exchange element and the skin (such Peltier thermal exchange element preferably being constructed in the manned disclosed in US Patent No. 6017337 by Pira) can be determined indirectly by measuring temperature difference between the cold side and hot side of the Peltier element, and the rate of flow of liquid cooling the heat sink on the hot side. Alternatively, a direct measurement of biofeedback (e.g. temperature) from a patient can be obtained by using sensor means to obtain a reading from a drainage catheter, which, for example, has been inserted into the body following knee reconstructive surgery. Although various preferred means of obtaining biofeedback readings have been disclosed herein, other means of obtaining such readings are not excluded.

In a particular embodiment, the thermal exchange device is a hydraulic device and/or a Peltier thermal exchange element.

The Pelier effect uses a thermoelectric cooling to create a heat flux between the junction of two different types of materials. A Peltier cooler, heater, or thermoelectric heat pump is a solid-state active heat-pump which transfers heat from one side of the device to the other side against the temperature gradient (from cold to hot), with consumption of electrical energy.

According to the present invention, a method for generating a feedback-driven protocol for thermal treatment of a human or animal body part is provided. The method comprising the steps of acquiring data of a thermal treatment from the body part and adapting the thermal treatment of the body part on the basis of the data acquired.

In a particular embodiment, the step of adapting the thermal treatment comprises monitoring the amount of thermal energy exchanged during the thermal treatment.

In a particular embodiment, the step of acquiring the data comprises determining a particular parameter of the body part.

In a particular embodiment, the method further comprises the step of documenting the adapting of the thermal treatment by generating a protocol and/or by selecting a known protocol.

A known protocol may be a protocol which was previously (that means before the present thermal treatment cycle of the body part) generated, for example, during a thermal treatment of a different patient and/or body part and/or during a thermal treatment of the same patient and/or body part but at a different time/date. Further, a known protocol may be a protocol which was generated by computer simulation particularly adapted to the disease of the patient.

For example, a (new) protocol is generated if it is determined that none of the known protocols "fits" with the actual needs of the patient. In particular, a protocol is generated on the basis of a known protocol (e.g. which was used in the cycle before) and then adapted depending on the data acquired from the present cycle.

In a particular embodiment, the adapting of the thermal treatment is an iteration process adapting the thermal treatment on the basis of the data acquired and data previously acquired.

The data previously acquired means data acquired before the present thermal treatment cycle of the body part. In doing so, the next treatment cycle may be further improved and adapted to the actual needs so that the recovery of the patient may be further enhanced.

According to the present invention, a computer program product is provided. The computer program product comprises one or more computer readable media having computer executable instructions for actuating and controlling the steps of the method as discussed above.

### BRIEF DESCRIPTION OF THE DRAWINGS

Fig. 1 schematically shows a thermal treatment device for use with a thermal exchange device;
Fig. 2 schematically shows a thermal treatment device for use with another thermal exchange device;
Fig. 3 is a graphical representation of the reduction in swelling by operation of the invention;
Fig. 4 is a graphical representation of the reduction in pain by operation of the invention; and
Fig. 5 is a graphical representation of the reduction in number of days the patient resides at the hospital or the number of days of inactivity of the patient.

### DETAILED DESCRIPTION OF THE INVENTION

Various embodiments of the present invention are described hereinafter with reference to the figures. It should be noted that the figures are not drawn to scale and elements of similar structures or functions are represented by like reference numerals throughout the figures. It should also be noted that the figures are only intended to facilitate the description of specific embodiments of the invention. In addition, an aspect described in conjunction with a particular embodiment of the present invention is not necessarily limited to that embodiment and can be practiced in any other embodiments of the present invention.

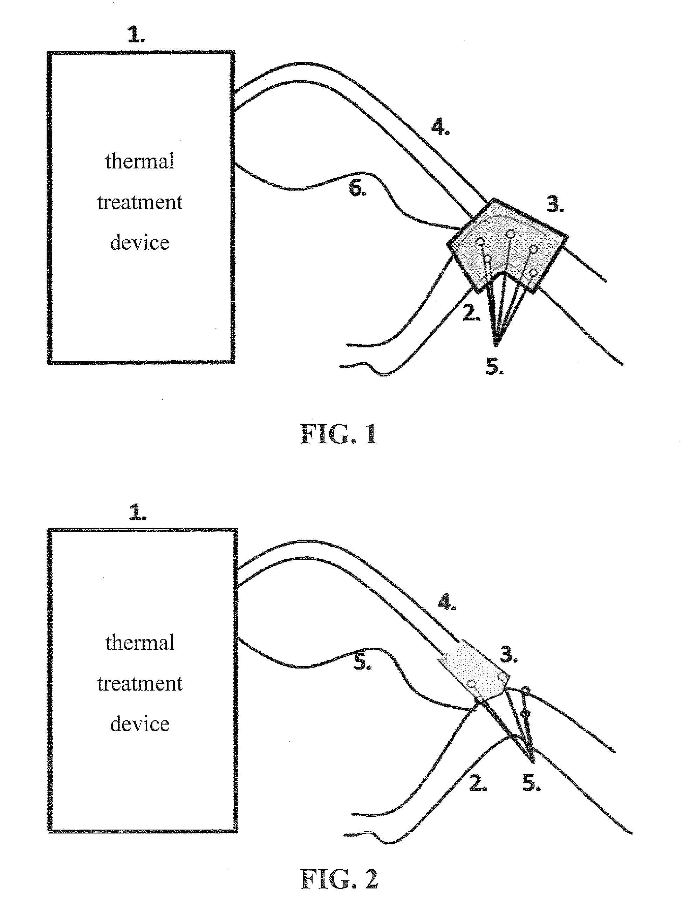

In Fig. 1 a thermal treatment device (1) controls the thermo-treatment of a part of a human body (2) by means of a thermal exchange device (3), capable of exchanging heat or cold. The thermal treatment device and the thermal exchange device may be connected by a hydraulic conduit, and electric conduit (providing a Peltier element), both or by other means. The effect of the heat exchange is measured and monitored through a measurement means (5) consisting of at least one sensor, sometimes combined with a signal condition and signal acquisition subsystem. The measurement means can be part of the thermal exchange device or it can be separate from it. The measurement means can be partially embedded in the device. The measurement means is adapted to measure the effect of the thermal exchange with the body part and/or measures aspects of the thermal energy exchange itself.

In Fig. 2 an embodiment is schematically shown, wherein the thermal exchange device (3) encompasses a Peltier thermal exchange element.

The effect of the thermal treatment represents a health benefit to the patient. This can manifest itself as a reduction of swelling, reduction of pain, rapid recovery, fewer days of residence at the hospital as well as other manifestations.

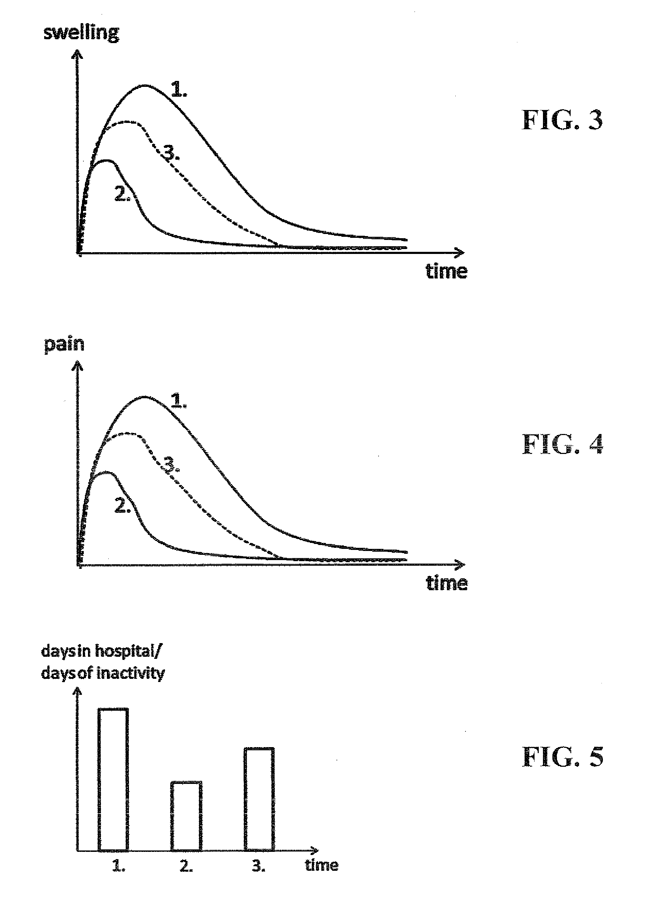

Fig. 3 represents an example where the thermo-treatment results in a reduction of swelling over time. At the leftmost point of Graph (1) an event occurs which causing swelling of a body part. After a certain time, if no thermal treatment is applied, a maximum swelling occurs, represented by the highest point in Graph (1). After that the swelling reduces slowly and eventually fades away completely. When optimal thermal treatment is applied, then the body part reacts differently to the same event. In Graph (2) thermal treatment is applied and results in a lower maximum point and a swelling reduction. In Graph (3) thermal treatment is applied in a suboptimal way: the maximum swelling is lower than in the case without treatment but higher than with optimal treatment. Swelling reduction occurs faster than without thermal treatment but slower than with it.
Fig. 4 depicts the same principle but applied on the health-beneficial effect of pain reduction. It can occur alone or in combination with the effect depicted in Fig. 3.
Fig. 5 depicts the same principle but expressed as a reduction in number of days the patient resides at the hospital or the number of days of inactivity of the patient. Bar (1) manifests itself without any thermo-treatment, bar (2) with the optimal thermo-treatment and bar (3) with sub-optimal thermo-treatment.

The present invention operates for example by enabling the thermal treatment device (1) to attain or approach the optimal treatment by gathering measurement data related to the patient's reaction to the on-going treatment or measurement data related to the thermal exchange with the body part and utilizing such data to adapt or select the optimal treatment protocols for the particular patient, in real-time.

The measurement data related to patient's reaction can be measured directly or indirectly, either using physical reactions to the treatment or psychological relations. Examples of measurements include measuring the dimensions of the body part (e.g. limb circumference), skin conductivity, skin color, skin temperature, skin texture, heart rate, blood pressure, blood saturation, or any other effects that may result from the thermo-treatment.

The measurement data can be measured directly or indirectly by monitoring the amount of energy exchanged by the thermal exchanger.

The measurement data may be relayed/transmitted to the thermal treatment device. The thermal treatment device takes the measurement data into account to optimize the on-going treatment protocol. The optimization entails optimizing (a) the temperature at which the thermal exchange takes place, (b) the pressure at which the thermal exchange takes place, (c) the duration of the thermal cycles, (d) the duration of the total treatment, (e) the temperature and pressure differences at various points in the treatment cycle. The effect of the optimization results in a beneficial effect to the patient, measured as either one or a combination of swelling reduction, pain reduction, fewer days in hospital, decreased inactivity time, better patient well-being, and any other beneficial effect that can be obtained through thermal treatment.

The optimization process combines the measurement data with the statistics gathered through medical research. At regular instances in time during the treatment the effect of the treatment resulting from the actual treatment parameters is correlated with known data. The treatment parameters are then adapted in a direction where better effect is expected. Preferably, following this adaptation new measurement data is collected and the above process is repeated until no additional treatment is required (thus providing an iteration process to optimize the thermal treatment).

While the invention has been illustrated and described in detail in the foregoing description, such illustration and description are to be considered illustrative or exemplary and non-restrictive; the invention is thus not limited to the disclosed embodiments. Features mentioned in connection with one embodiment described herein may also be advantageous as features of another embodiment described herein without explicitly showing these features. Variations to the disclosed embodiments can be understood and effected by those skilled in the art and practicing the claimed invention, from a study of the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures can not be used to advantage.

## Claims

1. Thermal treatment device for generating a feedback-driven protocol for thermal treatment of a human or animal body part, comprising
measurement means adapted to acquire data of a thermal treatment from the body part;
means for adapting the thermal treatment of the body part on the basis of the data acquired.

2. Device of claim 1, wherein the measurement means is adapted to determine the amount of thermal energy exchanged during the thermal treatment of the body part, on the basis of the acquired data.

3. Device of claim 1 or 2, wherein the means for adapting the thermal treatment is adapted to change the temperature and/or pressure and/or duration and/or duration/frequency of cycles of the thermal treatment and/or treatment content of each cycle of the thermal treatment.

4. Device of claim 1, 2 or 3, further comprising a storage means adapted to store the acquired data and to document the adapting of the thermal treatment.

5. Device of any one of the preceding claims, wherein the measurement means comprise at least one sensor adapted to determine a particular parameter of the body part.

6. Device of claim 5, wherein the particular parameter is at least one of the dimensions of the body part, the skin conductivity, the skin color, the skin temperature, the skin texture, the heart rate, the blood pressure, the blood saturation.

7. System for generating a feedback-driven protocol for thermal treatment of a human or animal body part, comprising:
a thermal treatment device of one of the preceding claims; and
a thermal exchange device connected to the thermal treatment device and adapted to provide a heating and/or a cooling of the body part.

8. System of claim 7, further comprising sensor means adapted to determine particular parameters of the body part.

9. System of claim 7 or 8, further comprising means for a controlled supply of medication, and wherein the system is preferably adapted to determine the response of the body part to the medication.

10. System of claim 7, 8, or 9, wherein the thermal exchange device is a hydraulic device and/or a Peltier thermal exchange element.

11. Method for generating a feedback-driven protocol for thermal treatment of a human or animal body part, comprising the steps of:
acquiring data of a thermal treatment from the body part;
adapting the thermal treatment of the body part on the basis of the data acquired.

12. Method of claim 11, wherein the step of adapting the thermal treatment comprises monitoring the amount of thermal energy exchanged during the thermal treatment.

13. Method of claim 10,11, or 12, wherein the step of acquiring the data comprises determining a particular parameter of the body part.

14. Method of claim 10, 11, 12 or 13, further comprising the step of:
documenting the adapting of the thermal treatment by generating a protocol and/or by selecting a known protocol.

15. Method of one of the preceding claims, wherein the adapting of the thermal treatment is an iteration process adapting the thermal treatment on the basis of the data acquired and data previously acquired.

16. Method of one of the preceding claims, further comprising a step of
updating the feedback-driven protocol by taking a response of the body part to a medical treatment into consideration.

17. Computer program product comprising one or more computer readable media having computer executable instructions for performing the steps of the method of any one of claims 10 to 14.
